# EUROPÄISCHE PATENTSCHRIFT

(11) **EP 2 768 420 B1**
(45) Veröffentlichungstag und Bekanntmachung des Hinweises auf die Patenterteilung: **28.12.2016**
(21) Anmeldenummer: 12780648.7
(22) Anmeldetag: 12.10.2012
(51) Int. Cl.: A61B 90/40, A61L 2/26

(54) **STERILEINPACKHILFE ZUM STERILEN EINPACKEN EINES GEGENSTANDES SOWIE KIT FÜR EINE STERILEINPACKHILFE**
STERILE PACKAGING AID FOR THE STERILE PACKAGING OF AN ARTICLE, AND KIT FOR A STERILE PACKAGING AID
AIDE AU CONDITIONNEMENT STÉRILE D'UN OBJET ET KIT D'AIDE AU CONDITIONNEMENT STÉRILE

(30) Priorität: 17.10.2011 DE 202011051648 U; 14.02.2012 DE 202012100490 U
(43) Veröffentlichungstag der Anmeldung: 27.08.2014
(73) Patentinhaber: Merete Innovations GmbH, 12247 Berlin (DE)
(72) Erfinder: ANAPLIOTIS, Emmanuel, 14195 Berlin (DE)
(74) Vertreter: Bittner, Thomas L.
(86) Internationale Anmeldenummer: PCT/DE2012/100320
(87) Internationale Veröffentlichungsnummer: WO 2013/056701

(56) Entgegenhaltungen:
- GB-A- 2 429 691
- US-A1- 2006 186 010
- US-A1- 2008 296 193

## Beschreibung

Die Erfindung betrifft eine Sterileinpackhilfe zum sterilen Einpacken eines Gegenstandes, insbesondere eines medizintechnischen Gegenstandes, sowie ein Kit für die Sterileinpackhilfe.

### Hintergrund der Erfindung

Eine derartige Einpackhilfe dient dazu, dem Benutzer das sterile Einpacken oder Abdecken eines Gegenstandes zu ermöglichen. Hierfür besteht beispielsweise Bedarf in Verbindung mit medizintechnischen Gegenständen wie Instrumenten oder Apparaten, die selbst nicht steril sind und auch nicht sterilisiert werden können. Um solche Apparate oder Instrumente der Medizintechnik trotzdem zum Beispiel im Umfeld einer chirurgischen Operation einsetzen zu können, bedarf es einer sterilen Verpackung des Apparates oder des Instrumentes. Bei einem solchen Gegenstand kann es sich beispielsweise um eine Fernbedienung eines im Operationsumfeld genutzten medizinischen Gerätes handeln.

Aus dem Dokument US 2008/0296193 A1 ist eine Klarsichtverpackung bekannt, die für verschiedene Gegenstände als Einpackhilfe genutzt werden kann. Die Verpackung besteht insbesondere aus einer durchsichtigen Schrumpffolie.

Aus dem Dokument DE 201 16 368 U1 ist ein Folienbeutel für medizinische Instrumente bekannt. Der sterile Folienbeutel ist einsetzbar zum sterilen Aufnehmen eines mobilen medizinischen Instrumentes oder Apparates. Zu diesem Zweck ist der Folienbeutel aus einem transparenten Material und mit einer versteiften Eingangsöffnung versehen. Durch die verschließbare Öffnung des Folienbeutels wird zum Verpacken des medizinischen Instrumentes oder Apparates ein Einsteck- oder Einführtrichter eingesteckt durch den hindurch dann der einzupackende Gegenstand in dem Folienbeutel eingebracht werden kann. Danach wird der Einführtrichter aus dem Folienbeutel wieder herausgezogen, worauf schließlich die Öffnung des Folienbeutels mit Hilfe einer Verschlusseinrichtung, die im Bereich des die Öffnung des Folienbeutels umgebenden Beutelrandes gebildet ist, verschlossen wird. Bei der bekannten Einpackhilfe besteht die Gefahr, dass beim Entfernen des Einführtrichters, nachdem also der steril einzupackende Gegenstand zunächst durch diesen in dem Folienbeutel eingeführt wurde, der Einführtrichter den Beutelrand mit der Verschlusseinrichtung berührt und hierdurch den Beutelrand selbst kontaminiert. Geschieht dieses zum Beispiel im Umfeld einer chirurgischen Operation, so kann es dann passieren, dass sich die Person, welche den Folienbeutel verschließt, selbst am Beutelrand kontaminiert, insbesondere die genutzten Operationshandschuhe. Es besteht deshalb Bedarf für eine verbesserte Sterileinpackhilfe.

### Zusammenfassung der Erfindung

Aufgabe der Erfindung ist es, eine verbesserte Sterileinpackhilfe sowie ein Kit für die Sterileinpackhilfe anzugeben, mit denen beim sterilen Einpacken oder Abdecken eines Gegenstandes Sterilität in verbessertem Umfang gewährleistet ist.

Diese Aufgabe wird erfindungsgemäß durch eine Sterileinpackhilfe zum sterilen Einpacken eines Gegentandes nach dem unabhängigen Anspruch 1 gelöst. Vorteilhafte Ausgestaltungen der Erfindung sind Gegenstand von abhängigen Unteransprüchen.

Mit Hilfe der vorgeschlagenen Sterileinpackhilfe wird insbesondere sichergestellt, dass es beim sterilen Einpacken oder Verpacken des Gegenstandes nicht zur unbeabsichtigten Kontamination, also dem Verlust der Sterilität, im Bereich des Beutelrandes kommt, welcher die Beutelöffnung des Folienbeutels umgibt. Dieses wird insbesondere dadurch verhindert, dass der die Beutelöffnung umgebende Beutelrand mit Hilfe der Schutzabdeckung innen- und außenseitig bedeckt ist.

Es ist insbesondere erreicht, dass die Schutzabdeckung, mit der der Beutelrand zum sterilen Einführen des einzupackenden Gegenstandes beim Einführen desselben in den sterilen Folienbeutel abgedeckt wird, hierbei sicher auf dem Beutelrand verbleibt und sich nicht versehentlich vom Beutelrand löst, was das sterile Einpacken behindern oder sogar unmöglich machen würde.

Nach einem Aspekt ist das unbeabsichtigte Lösen der auf dem Beutelrand angeordneten Schutzabdeckung, insbesondere beim Herausziehen eines zuvor beim Einführen des zu verpackenden Gegenstandes genutzten Einführtrichters, insbesondere dadurch verhindert, dass an der Schutzabdeckung im Bereich von deren Außenabdeckung ein oder mehrere Fingereingriff-Ausnehmungen gebildet sind. Derartige Fingereingriff-Ausnehmungen können auch mit der Haftverbindung kombiniert werden.

Nachfolgend werden bevorzugte Ausgestaltungen erläutert, die bei der Sterileinpackhilfe vorgesehen sein können. Die Erfindung wird im Anspruch 1 definiert und weitere Ausführungsformen finden sich in den abhängigen Ansprüchen.

Nach dem Einführen oder Einstecken des steril aufzunehmenden Gegenstandes durch den Einführtrichter hindurch in dem Folienbeutel wird der Einführtrichter beim Verpacken des Gegenstandes herausgezogen. Zu diesem Zeitpunkt verbleibt die Schutzabdeckung bevorzugt noch an dem Folienbeutel, d. h. der die Beutelöffnung umgebende Rand wird nach wie vor von der Schutzabdeckung umgriffen und somit geschützt. Hierbei sichert insbesondere die Haftverbindung die Schutzabdeckung gegen ein unbeabsichtigtes Lösen vom Folienbeutel. Nachdem der Einführtrichter herausgezogen ist, kann nun auch die Schutzabdeckung entfernt werden. Hierbei hält der Nutzer den Folienbeutel fest, vorteilhafter Weise derart, dass auch der eingesteckte Gegenstand gehalten wird. Nach dem Entfernen der Schutzabdeckung ist der die Beutelöffnung umgebende Beutelrand freigegeben, so dass nun die Verschlusseinrichtung zum Verschließen des Folienbeutels mit dem eingesteckten Gegenstand benutzt werden kann. Vorteilhafterweise kann vorgesehen sein, dass die Schutzabdeckung, wenn sie an dem Folienbeutel angeordnet ist, auch die Verschlusseinrichtung selbst mit abdeckt, wodurch der Schutz gegen eine unbeabsichtigte Kontamination weiter verbessert ist. Diese vorgeschlagene Sterileinpackhilfe ermöglicht es insbesondere medizinischem Personal beim Abdecken oder Verpacken eines Gegenstandes im Reinraumumfeld, also zum Beispiel in einem Operationssaal, die eigene Sterilität aufrechtzuerhalten, insbesondere dadurch, dass eine Kontamination der benutzten Schutzhandschuhe beim Verpacken des Gegenstandes verhindert ist. Beim Verschließen des Folienbeutels, nachdem Einführtrichter und Schutzabdeckung entfernt wurden, kommt es nicht zur Kontamination der Fingerhandschuhe, die mit dem Beutelrand beim Verschließen der Verschlusseinrichtung in Kontakt kommen.

Es kann vorgesehen sein, das die Schutzabdeckung und / oder der Einführtrichter in Oberflächenabschnitten mit Benutzerhinweisen bedruckt sind, zum Beispiel Gebrauchsanweisungen.

Eine bevorzugte Weiterbildung der Erfindung sieht vor, dass sich der Einführtrichter in dem Folienbeutel bis zum Beutelboden erstreckt. Hierbei kann vorgesehen sein, dass der Einführtrichter im vollständig in den Folienbeutel eingesteckten Zustand die innenseitige Beuteloberfläche im Wesentlichen vollständig erfassend abdeckt. Der in den Folienbeutel einzusteckende Gegenstand gleitet dann beim Einführen oder Einstecken im Wesentlichen vollständig auf der Innenseite des Einführtrichters zum Boden oder Grund des sterilen Folienbeutels.

Bei einer zweckmäßigen Ausgestaltung der Erfindung kann vorgesehen sein, dass der Einführtrichter im vollständig eingeführten Zustand über den Beutelrand nach außen übersteht. Ein solcher Überstand oberhalb des die Beutelöffnung umgebenden Beutelrandes des Folienbeutels erleichtert die Handhabung des Einführtrichters, insbesondere beim Herausziehen des Einführtrichters durch den Benutzer im Operationsraum nach dem Einstecken des einzupackenden Gegenstandes. Der Einführtrichter kann hierbei im Bereich des Überstandes mit den Fingern gegriffen und herausgezogen werden, ohne die restliche Sterileinpackhilfe zu berühren und zu kontaminieren.

Eine vorteilhafte Ausführungsform der Erfindung sieht vor, dass die Außenabdeckung außen auf dem Folienbeutel einen Griffbereich gebildet ist, in welchem ein steriler Nutzer den Folienbeutel fassen und selbst entfernen kann. Der Griffbereich ermöglicht es dem Nutzer zum Beispiel, nach dem Herausziehen des Einführtrichters den Folienbeutel in diesem Bereich und hierdurch auch den eingeführten Gegenstand zwischen den Fingern festzuhalten, wenn die Schutzabdeckung vom Folienbeutel entfernt wird.

Bevorzugt sieht eine Fortbildung der Erfindung vor, dass der Griffbereich wenigstens teilweise mittels einer Griffausnehmung an der Außenabdeckung gebildet ist. Die Griffausnehmung kann in einer Ausführungsform im Randbereich der Außenabdeckung, zum Beispiel im zur Beutelöffnung distalen Randbereich der Außenabdeckung gebildet sein, wodurch es erleichtert ist, einen möglichst großen Bereich der Außenfläche des Folienbeutels mit der Außenabdeckung zu bedecken.

Bei einer vorteilhaften Ausgestaltung der Erfindung kann vorgesehen sein, dass die Innenabdeckung als eine Abdecklippe gebildet ist, die auf der Beutelinnenseite auf den die Beutelöffnung umgebenden Beutelrand begrenzt ist.

Bei einer vorteilhaften Ausgestaltung der Erfindung kann vorgesehen sein, dass die Schutzabdeckung und / oder der Einführtrichter aus einem Material aus der folgenden Gruppe bestehen: Papier, Karton und Pappe.

Eine bevorzugte Weiterbildung der Erfindung sieht vor, dass der Folienbeutel wenigstens teilweise aus einer Klarsichtfolie besteht. Klarsichtfolie unterstützt eine gute Sichtbarkeit des Gegenstandes in dem Folienbeutel von außen, was zum Beispiel in Verbindung mit steril zu verpackenden Gegenständen von besonderer Bedeutung ist, die Bedienfelder aufweisen, welche vom Benutzer durch den Folienbeutel hindurch betätigt werden sollen. Beispielsweise ist dieses der Fall bei Fernbedienungen von medizinischen Geräten, einem Smartphone oder eines Touch-Tablettcomputers, die mittels der Einpackhilfe steril verpackt werden. Klarsichtfolienabschnitte sind aber auch von Vorteil, wenn der eingepackte Gegenstand ein Display aufweist, welches durch den Folienbeutel hindurch einsehbar sein soll. Beispielsweise kann vorgesehen sein, ein sogenanntes Smartphone oder einen Tablettcomputer steril einzupacken. Auch hier ist es von Vorteil, wenn mit Hilfe der Klarsichtfolie das Display einsehbar ist, zum Beispiel für das Abrufen und Betätigen von bestimmten Applikationen.

Bei einer zweckmäßigen Ausgestaltung der Erfindung kann vorgesehen sein, dass am Folienbeutel eine der Beutelöffnung zugeordnete Verschlusseinrichtung gebildet ist. Die Verschlusseinrichtung kann beispielsweise mit einem Haft- oder Adhäsionsverschluss oder mit einem Druck- oder Ziehverschluss (Reißverschluss) gebildet sein. Vorzugsweise ist die Verschlusseinrichtung im Bereich des Beutelrandes gebildet, die die Beutelöffnung umgibt. Die Schutzabdeckung schützt dann insbesondere auch die Verschlusseinrichtung gegen Kontamination beim Einführen des steril zu verpackenden Gegenstandes.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1: eine schematische Darstellung einer Schutzabdeckung,
- Fig. 2: eine perspektivische Darstellung einer weiteren Ausführungsform einer Schutzabdeckung,
- Fig. 3: eine schematische Darstellung eines Einführ- oder Einstecktrichters,
- Fig. 4: eine schematische Darstellung eines Folienbeutels,
- Fig. 5: eine schematische Darstellung einer Sterileinpackhilfe, bei der zunächst der Einführtrichter in den Folienbeutel eingesteckt ist,
- Fig. 6: eine schematische Darstellung der Einführpackhilfe nach Fig. 5, wobei nun zusätzlich die Schutzabdeckung an dem Folienbeutel angeordnet ist,
- Fig. 7: eine perspektivische Darstellung einer anderen Ausführungsform einer Schutzabdeckung, die Oberflächenbereiche aufweist, welche mit einem Haftmittel versehen sind,
- Fig. 8: eine perspektivische Darstellung einer weiteren Ausführungsform einer Schutzabdeckung, bei der auf einer Außenseite ein Haftetikett angebracht ist,
- Fig. 9: eine schematische Darstellung der Schutzabdeckung aus Fig. 1, wobei eine Außenabdeckung Fingergriff-Ausnehmungen aufweist, und
- Fig. 10: eine perspektivische Darstellung einer weiteren Ausführungsform einer Schutzabdeckung, bei der die Außenabdeckung ebenfalls Fingergriff-Ausnehmungen aufweist.

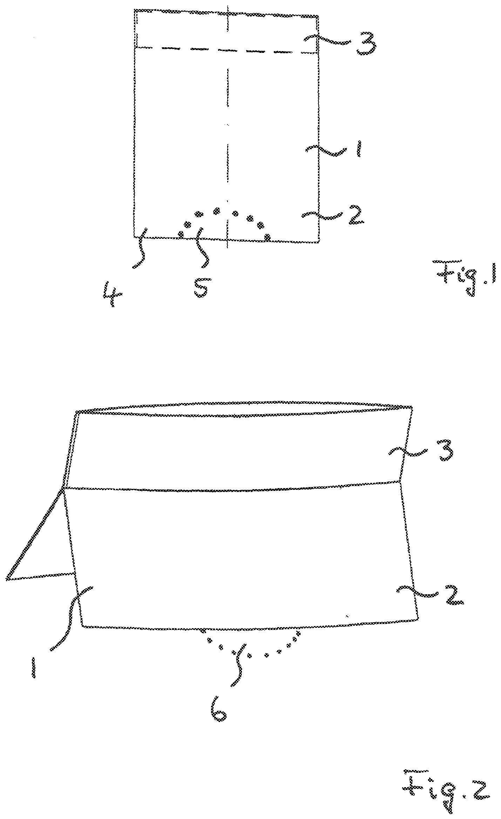

Die Fig. 1 bis 4 zeigen schematische Darstellungen von Elementen einer Sterileinpackhilfe. Fig. 1 zeigt eine schematische Darstellung einer Schutzabdeckung 1, die mit einer Außenabdeckung 2 und einer hieran gebildeten und nach innen umgeschlagenen Innenabdeckung 3 ausgeführt ist, wobei die Innenabdeckung 3 in dem dargestellten Ausführungsbeispiel als Abdecklippe gebildet ist, die nach Innen umgeschlagen ist. In einer Ausführungsform ist bei der Schutzabdeckung 1 die Außenabdeckung 2 als ein Ring gebildet, an dem innenseitig umgeklappt die Innenabdeckung 3 umläuft.

Gemäß Fig. 1 kann die Schutzabdeckung 1 in einer Ausführungsform in einem unteren Randbereich 4 eine Griffaussparung 5 aufweisen, was in Fig. 1 mittels einer gepunkteten Linie angedeutet ist.

Fig. 2 zeigt eine perspektivische Darstellung einer weiteren Ausführungsform einer Schutzabdeckung 1, wobei für gleiche Merkmale die gleichen Bezugszeichen wie in Fig. 1 verwendet werden. Bei der Ausführung in Fig. 2 ist eine Grifflasche 6 an der Außenabdeckung 2 mittels gestrichelter Linie angedeutet. In einer Ausgestaltung können die Griffaussparung 5 und die Grifflasche 6 kombiniert werden, zum Beispiel mittels Ausbilden auf der jeweils anderen Seite der Außenabdeckung 2.

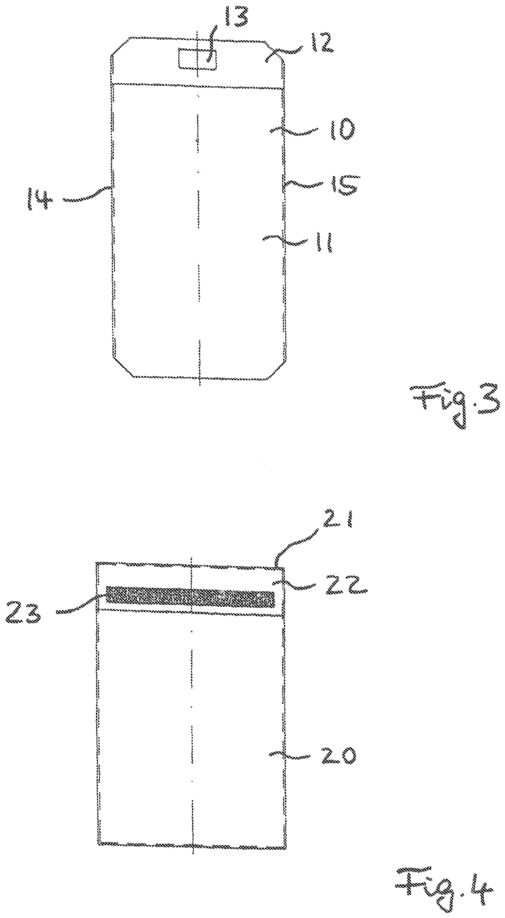

Fig. 3 zeigt eine schematische Darstellung eines Einsteck- oder Einführtrichters 10, bei dem in der dargestellten Ausführungsform ein Trichterabschnitt 11 und Griffabschnitt 12 mit einer Öffnung 13 gebildet sind.

Fig. 4 zeigt eine schematische Darstellung eines Folienbeutels 20 mit einer Beutelöffnung 21, die von einem Beutelrand 22 umgeben ist, indem eine Verschlusseinrichtung 23 gebildet ist, beispielsweise mit einem Haftverschluss.

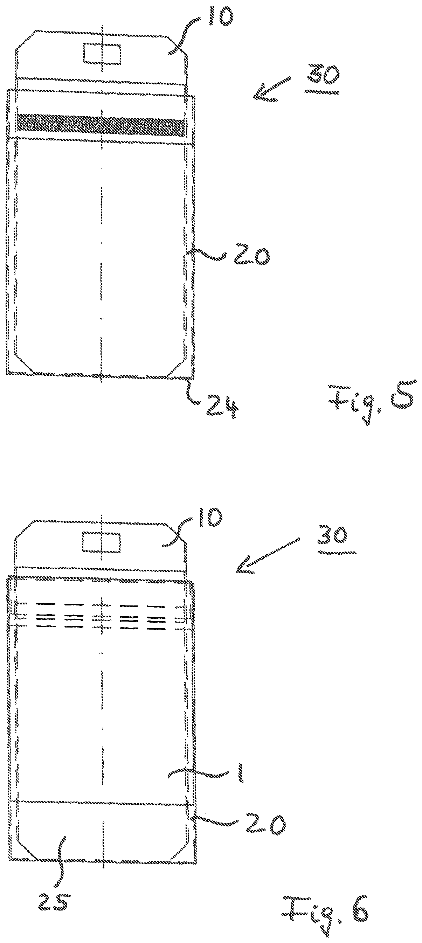

Die in den Fig. 1 bis 4 dargestellten Elemente können nun zum Ausbilden einer Sterileinpackhilfe verwendet werden, was im Folgenden unter Bezugnahme auf die Fig. 5 und 6 näher erläutert wird. Fig. 5 zeigt eine schematische Darstellung einer Sterileinpackhilfe 30, zu deren Ausbildung zunächst der Einstecktrichter 10 durch die Beutelöffnung 21 in den Folienbeutel 20 eingeführt wird, derart, dass sich der Einstecktrichter 10 im Inneren des Folienbeutels 20 bis zum Beutelgrund 24 hin erstreckt. Bei der dargestellten Ausführungsform kleidet der Einstecktrichter 10 auf diese Weise den Folienbeutel 20 im Wesentlichen vollständig innenseitig aus. Es kann alternativ vorgesehen sein, dass der Einstecktrichter 10 in seitlichen Randbereichen 14, 15 (vgl. Fig. 3) aufgeschlitzt ist. Hierdurch wären dann gegebenenfalls Teile der Innenseite des Folienbeutels 20 nicht von dem Einführtrichter 10 abgedeckt.

Zur vollständigen Ausbildung der Sterileinpackanordnung 30 wird nun gemäß Fig. 6 noch die Schutzabdeckung 1 in den Folienbeutel 20 eingeführt, derart, dass im Bereich des Beutelrandes 22 die lippenartig ausgeführte Innenabdeckung 3 zwischen Außenseite des Einführtrichters 10 und Beutelrand 22 angeordnet ist. Die Außenabdeckung 2 liegt außen auf dem Folienbeutel 20, so dass der Beutelrand 22 mit der hier gebildeten Verschlusseinrichtung 23 umgegriffen und gegen Kontamination geschützt ist. Auf der äußeren Oberfläche des Folienbeutels 20 ist ein Griffbereich 25 (vgl. Fig. 6) freigelassen.

Die Sterileinpackhilfe kann nun zum Einführen des aufzunehmenden Gegenstandes genutzt werden, indem dieser durch den Einführtrichter 10 hindurch in dem Folienbeutel 20 eingebracht wird. Hierauf wird der Einführtrichter 10 wieder herausgezogen, derart, dass der zu verpackende Gegenstand in dem Folienbeutel 20 verbleibt. Anschließend wird noch die Schutzabdeckung 1 von dem Folienbeutel 20 abgenommen, wobei der Nutzer hierbei den Folienbeutel 20 mit den Fingern festhält. Durch das Abnehmen der Schutzabdeckung 1 wird der Beutelrand 22 mit der Verschlusseinrichtung 23 freigegeben, und zwar unter Aufrechterhaltung der Sterilität des Folienbeutels 20 auch in diesem Bereich, so dass dann der Folienbeutel 20 mit dem hierin aufgenommenen Gegenstand vom Nutzer verschlossen werden kann.

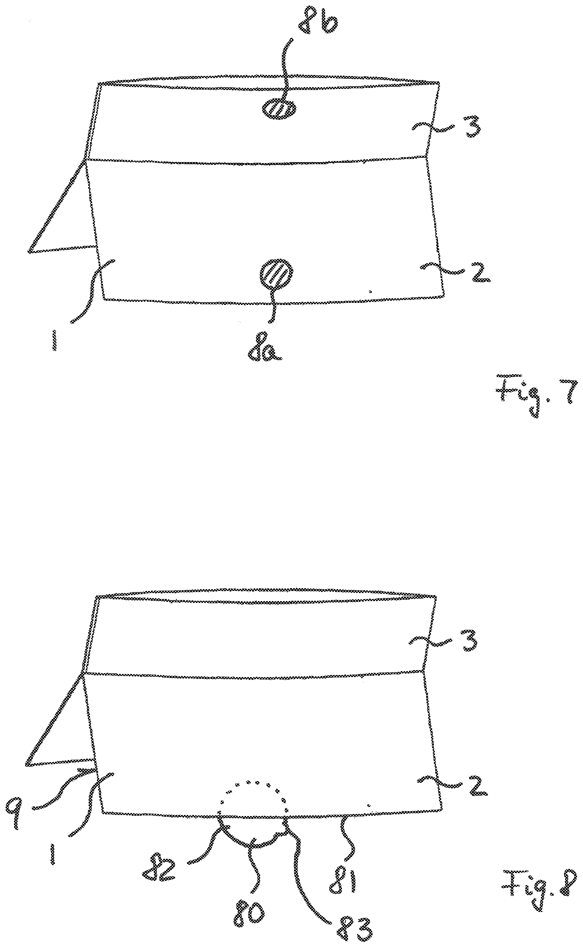

Fig. 7 und 8 zeigen jeweils eine perspektivische Darstellung einer anderen Ausführungsform einer Schutzabdeckung 1, wobei für gleiche Merkmale die gleichen Bezugszeichen wie in den Fig. 1 und 2 verwendet werden.

Bei der Ausführung in Fig. 7 verfügt die Schutzabdeckung 1 im Bereich sowohl der Außenabdeckung 2 als auch der Innenabdeckung 3 über Haftflächen 8a, 8b, in denen ein Haftmittel, beispielsweise eine Klebematerial, auf die entsprechende Oberfläche aufgebracht ist, sodass die Schutzabdeckung 1 beim Anbringen auf dem Beutelrand 22 des Folienbeutels 20 hierin haftend befestigt werden kann, indem die Schutzabdeckung 1 im Bereich der Haftflächen 8a, 8b an die Beuteloberfläche gedrückt wird. Abweichend von der Ausführungsform in Fig. 7 können Haftmittelbereiche auch nur an der Außenabdeckung 2 oder nur an der Innenabdeckung 3 vorgesehen sein. Auch können ein oder mehrere Haftflächen jeweils an der Außenabdeckung 2 und / oder der Innenabdeckung 3 vorgesehen sein.

Fig. 8 zeigt eine perspektivische Darstellung einer weiteren Ausgestaltung einer Schutzabdeckung 1, bei der auf einer Außenseite 9 der Außenabdeckung 2 ein Haftetikett 80 angebracht ist, welches am Rand 81 übersteht, sodass die Schutzabdeckung 1 beim Anbringen auf dem Beutelrand 22 mit Hilfe des überstehenden Abschnittes 82 des Haftetiketts 80 lösbar auf der Außenseite des Folienbeutels 20 befestigt werden kann, um die Schutzabdeckung 1 beim Einführen des zu verpackenden Gegenstandes an dem Folienbeutel 20 zu sichern. Hierzu wird mit Hilfe des Haftetiketts 80 eine lösbare Haftverbindung hergestellt.

Gemäß Fig. 8 ist an dem Haftetikett 80 ein Griffabschnitt 83 gebildet, der frei von Haftmittel ist und mit den Fingern gegriffen werden kann, um an dem Haftetikett zu ziehen.

Alternativ oder ergänzend kann ein vergleichbares Haftetikett (nicht dargestellt) auf der Innenabdeckung 3 gebildet sein. Auch mehrere Haftetiketten können jeweils auf der Innen- und oder der Außenabdeckung 3, 2 angeordnet sein. Weiterhin sind die eine oder die mehreren Haftetiketten auch kombinierbar mit einer oder mehreren Haftflächen.

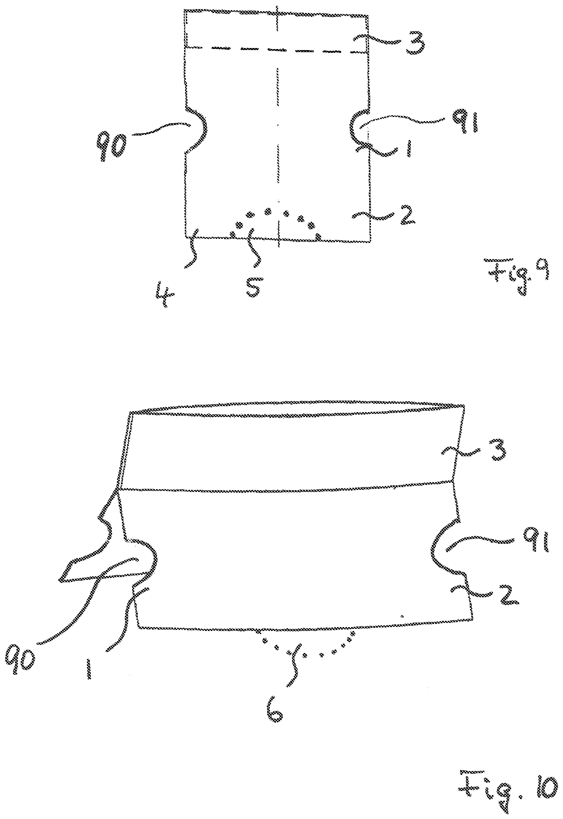

Die Fig. 9 und 10 zeigen weitere Ausgestaltungen für eine Schutzabdeckung 1, die mit der Außen- und der Innenabdeckung 2, 3 versehen ist. Im Bereich der Außenabdeckung 2 sind Fingereingriff-Ausnehmungen 90, 91 vorgesehen, die es dem Benutzer ermöglichen, mittels Fingern den Folienbeutel 20 sowie den hierin aufgenommenen Einstecktrichter 10 seitlich zu greifen, insbesondere um hierdurch die Einführöffnung des Einführtrichters 10 zu bilden, durch welche hindurch dann der zu verpackende Gegenstand in den Folienbeutel 20 eingeführt werden kann. Darüber hinaus ermöglichen es die Fingereingriff-Ausnehmungen 90, 91 den Einführtrichter 10 mittels der Finger noch festzuhalten, während die Schutzabdeckung 1 nach dem Einführen des zu verpackenden Gegenstandes vom Beutelrand 22 nach oben weggeführt wird. Auf diese Weise ist verhindert, dass hierbei die Schutzabdeckung 1 unbeabsichtigt zusammen mit dem Einführtrichter 10 herausgezogen wird, was zur Kontamination des Folienbeutels 20, insbesondere im Beutelrandbereich 22, führen könnte. Die FingereingriffAusnehmungen 90, 91 ermöglichen das Halten der Einpackhilfe mit einer Hand während des gesamten Einpackprozesses.

Die Fig. 9 und 10 zeigen lediglich eine mögliche Ausführungsform der Ausprägung der Fingereingriff-Ausnehmungen 90, 91. Es kann vorgesehen sein, die FingereingriffAusnehmungen auch nur auf einer Seite der Außenabdeckung 1 vorzusehen. Auch können an einem Flügel der Außenabdeckung 2 mehr als zwei Fingereingriff-Ausnehmungen angeordnet sein, was den Eingriff weiterer Finger derselben Hand ermöglicht.

Bei der in den Fig. 9 und 10 dargestellten Ausführungsform oder anderen Ausgestaltungen ist die Länge des Folienbeutels 20 vorzugsweise größer als die Erstreckung des Einführtrichters 10 in den Folienbeutel 20 hinein. Soweit die Fingereingriff-Ausnehmungen 90, 91 bei einer Ausgestaltung der Sterileinpackhilfe vorgesehen sind, erstreckt sich der Einführtrichter 10 im eingesteckten Zustand wenigstens bis zu den oder über die Fingereingriff-Ausnehmungen 90, 91 hinaus, sodass in deren Bereich der Einführtrichter 10 mittels zweier Finger seitlich gegriffen werden kann.

Gemäß Fig. 9 sind auf der Außenabdeckung 2 Hinweispfeile A für den Benutzer, die auf die Nutzung der Fingereingriff-Ausnehmungen 90, 91 hinweisen. Diese Hinweise können optional ergänzt werden durch einen sprachlichen Hinweis, beispielsweise die Formulierung "Press to open".

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Sterileinpackhilfe zum sterilen Einpacken eines Gegenstandes, insbesondere eines medizintechnischen Gegenstandes, bei der eine Einpackanordnung (30) gebildet ist mit
- einem sterilen Folienbeutel (20) mit einer verschließbaren Beutelöffnung (21), und
- einem Einführtrichter (10), der durch die Beutelöffnung (21) mit dem von einer Schutzabdeckung (1) umgriffenen Beutelrand (22) hindurch lösbar in den Folienbeutel (20) eingesteckt ist und durch den hindurch ein im Folienbeutel (20) aufzunehmender Gegenstand in dem Folienbeutel (20) einführbar ist,
wobei die Schutzabdeckung (1) mit einer Außenabdeckung (2) sowie einer hieran gebildeten Innenabdeckung (3) ausgeführt und lösbar an dem Folienbeutel (20) angeordnet ist, derart, dass die Außenabdeckung (2) den Folienbeutel (20) außen von der Beutelöffnung (21) ausgehend teilweise bedeckt und dass ein Beutelrand (22) im Bereich der Beutelöffnung (21) umgriffen wird, indem die Innenabdeckung (3) nach innen und in die Beutelöffnung (21) hinein umgeschlagen ist, wobei die Außenabdeckung (2) der Schutzabdeckung (1) eine oder mehrere Fingergriff-Ausnehmungen (90, 91) aufweist.

2. Sterileinpackhilfe nach Anspruch 1, **dadurch gekennzeichnet, dass** sich der Einführtrichter (10) in dem Folienbeutel (20) bis zum Beutelboden (24) erstreckt.

3. Sterileinpackhilfe nach Anspruch 1 oder 2, **dadurch gekennzeichnet, dass** der Einführtrichter (10) im vollständig eingeführten Zustand über den Beutelrand (22) nach außen übersteht.

4. Sterileinpackhilfe nach mindestens einem der vorangehenden Ansprüche, dadurch g e - **kennzeichnet,** dass die Außenabdeckung (2) außen auf dem Folienbeutel (20) einen Griffbereich (25) gebildet ist, in welchem ein Nutzer den Folienbeutel (20) fassen kann.

5. Sterileinpackhilfe nach Anspruch 4, **dadurch gekennzeichnet, dass** der Griffbereich (25) wenigstens teilweise mittels einer Griffausnehmung (5) an der Außenabdeckung (2) gebildet ist.

6. Sterileinpackhilfe nach mindestens einem der vorangehenden Ansprüche, dadurch **ge- kennzeichnet,** dass die Innenabdeckung (3) als eine Abdecklippe gebildet ist, die auf der Beutelinnenseite auf den die Beutelöffnung (21) umgebenden Beutelrand (22) begrenzt ist.

7. Sterileinpackhilfe nach mindestens einem der vorangehenden Ansprüche, dadurch **ge- kennzeichnet,** dass die Schutzabdeckung (1) und / oder der Einführtrichter (10) aus einem Material aus der folgenden Gruppe bestehen: Papier, Karton und Pappe.

8. Sterileinpackhilfe nach mindestens einem der vorangehenden Ansprüche, dadurch **ge- kennzeichnet,** dass der Folienbeutel (20) wenigstens teilweise aus einer Klarsichtfolie besteht.

9. Sterileinpackhilfe nach mindestens einem der vorangehenden Ansprüche, dadurch **ge- kennzeichnet,** dass am Folienbeutel (20) eine der Beutelöffnung (21) zugeordnete Verschlusseinrichtung (23) gebildet ist.

## Claims

1. A sterile packaging aid for the sterile packaging of an object, in particular a medical-technology object, where a packaging arrangement (30) is formed with
- a sterile film bag (20) with a closable bag opening (21), and
- an insertion funnel (10) which is detachably inserted into the film bag (20) through the bag opening (21) having a bag rim (22) embraced by the protective covering (1), and through which an object to be received in the film bag (20) can be inserted into the film bag (20),
wherein the protective covering (1) is implemented with an outer covering (2) as well as an inner covering (3) formed thereon and detachably arranged on the film bag (20), such that the outer covering (2) partially covers the film bag (22) starting from the bag opening (21) and in that a bag rim (22) in the area of the bag opening (21) is embraced in that the inner covering (3) is folded over inwards and into the interior of the bag opening (21), wherein the outer covering (2) of the protective covering (1) comprises one or more finger grip recesses (90, 91).

2. The sterile packaging aid according to claim 1, **characterised in that** the insertion funnel (10) extends into the interior of the film bag (20) as far as the bag floor (24).

3. The sterile packaging aid according to claim 1 or 2, **characterised in that** the insertion funnel (10), when fully inserted, protrudes towards the outside over the bag rim (22).

4. The sterile packaging aid according to at least one of the preceding claims, **characterised in that** the outer covering (2) on the outside of the film bag (20) forms a grip area (25), in which a user can grasp the film bag (20).

5. The sterile packaging aid according to claim 4, **characterised in that** the grip area (25) is formed at least partially by a grip recess (5) on the outer covering (2).

6. The sterile packaging aid according to at least one of the preceding claims, **characterised in that** the inner covering (3) is configured as a covering lip, which on the inside of the bag is limited to the bag rim (22) surrounding the bag opening (21).

7. The sterile packaging aid according to at least one of the preceding claims, **characterised in that** the protective covering (1) and/or the insertion funnel (10) consist of a material from the following group: paper, carton or cardboard.

8. The sterile packaging aid according to at least one of the preceding claims, **characterised in that** the film bag (20) consists at least partially of a transparent film.

9. The sterile packaging aid according to at least one of the preceding claims, **characterised in that** a closure device (23) associated with the bag opening (21) is formed on the film bag (20).

## Revendications

1. Aide au conditionnement stérile pour le conditionnement stérile d'un objet, en particulier d'un objet médicotechnique, dans lequel un système de conditionnement (30) est formé avec :
- un sachet en feuille stérile (20) avec une ouverture de sachet refermable (21), et
- un entonnoir d'introduction (10) qui est inséré à travers l'ouverture de sachet (21), avec le bord de sachet (22) entouré par une couverture de protection (1), de manière détachable dans le sachet en film (20) et à travers lequel un objet à réceptionner dans le sachet en film (20) peut être introduit dans le sachet en film (20),
dans lequel la couverture de protection (1) est réalisée avec une couverture extérieure (2) ainsi qu'une couverture intérieure (3) formée sur celle-ci et est disposée de façon détachable sur le sachet en feuille (20) de manière à ce que la couverture extérieure (2) recouvre partiellement le sachet en feuille (20) à l'extérieur en partant de l'ouverture de sachet (21) et qu'un bord de sachet (22) soit entouré dans la zone de l'ouverture de sachet (21) en ce que la couverture intérieure (3) est rabattue vers l'intérieur et jusqu'à l'intérieur de l'ouverture de sachet (21), dans laquelle la couverture extérieure (2) de la couverture de protection (1) présente un ou plusieurs évidements de prise de doigts (90, 91).

2. Aide au conditionnement stérile selon la revendication 1, **caractérisée en ce que** l'entonnoir d'introduction (10) s'étend dans le sachet en feuille (20) jusqu'au fond du sachet (24).

3. Aide au conditionnement stérile selon la revendication 1 ou 2, **caractérisé en ce que** l'entonnoir d'introduction (10) est en saillie vers l'extérieur du bord de sachet (22) à l'état complètement introduit.

4. Aide au conditionnement stérile selon l'une au moins des revendications précédentes, **caractérisée en ce que** la couverture extérieure (2) est formée à l'extérieur sur le sachet en feuille (20) avec une zone de prise (25) dans laquelle un utilisateur peut saisir le sachet en feuille (20).

5. Aide au conditionnement stérile selon la revendication 4, **caractérisée en ce que** la zone de saisie (25) est formée au moins partiellement au moyen d'un évidement de saisie (5) sur la couverture extérieure (2).

6. Aide au conditionnement stérile selon l'une au moins des revendications précédentes, **caractérisée en ce que** la couverture intérieure (3) est réalisée en tant qu'une lèvre de recouvrement, laquelle est limitée sur le côté intérieur de sachet au bord du sachet (22) entourant l'ouverture de sachet (21).

7. Aide au conditionnement stérile selon l'une au moins des revendications précédentes, **caractérisée en ce que** la couverture de protection (1) et/ou l'entonnoir d'introduction (10) se composent d'un matériau du groupe suivant : du papier, du carton et du carton-pâte.

8. Aide au conditionnement stérile selon l'une au moins des revendications précédentes, **caractérisée en ce que** le sachet en feuille (20) se compose au moins partiellement d'une feuille transparente.

9. Aide au conditionnement stérile selon l'une au moins des revendications précédentes, **caractérisée en ce qu'**un dispositif de fermeture (23) associé à l'ouverture de sachet (21) est formé sur le sachet en feuille (20).
